# EUROPÄISCHE PATENTSCHRIFT

(11) **EP 1 725 330 B1**
(45) Veröffentlichungstag und Bekanntmachung des Hinweises auf die Patenterteilung: **13.02.2008**
(21) Anmeldenummer: 04802746.0
(22) Anmeldetag: 17.11.2004
(51) Int. Cl.: B01L 3/00, G01N 33/53, B29C 35/08, B29C 59/04, G03F 7/20

(54) **MIKROFLUIDIK-CHIP**
MICROFLUIDIC CHIP
PUCE MICROFLUIDIQUE

(30) Priorität: 17.03.2004 DE 102004013161
(43) Veröffentlichungstag der Anmeldung: 29.11.2006
(73) Patentinhaber: microTec Gesellschaft für Mikrotechnologie mbH, 47057 Duisburg (DE)
(72) Erfinder: Götzen, Reiner, 47239 Duisburg (DE)
(74) Vertreter: Röther, Peter
(86) Internationale Anmeldenummer: PCT/DE2004/002533
(87) Internationale Veröffentlichungsnummer: WO 2005/089944

(56) Entgegenhaltungen:
- WO-A1-00/58724
- WO-A1-98/44072
- DE-A1- 4 420 996
- DE-A1- 10 144 579
- US-A1- 2002 125 612
- US-B1- 6 213 151
- US-B1- 6 623 613
- [Online] XP002324492 MEDICA.de News-Archiv Gefunden im Internet: URL:http://www16.medica.de/cipp/md_medica/ custom/pub/content,lang,1/oid,11158/ticket ,g_u_e_s_t/local_lang,1> & [Online] Gefunden im Internet: URL:http://www.microtec-d.com/media/a_home page/a01_startseite/produkte_biogentechnik .pdf>
- GOETZEN R ET AL: "Rapid Product Development RMPD Schusseltechnologie fur die Aufbau- und Verbindungstechnik von Mikrosystemen"[Online] 25. Juli 2002 (2002-07-25), XP002207923 Gefunden im Internet: URL:www.microtec-d.com/media/e_downloads/m icrotecneu.pdf>

## Beschreibung

Die Erfindung betrifft einen Mikrofluidik-Chip für biologische, chemische und medizinische Analytik und Synthese, in dem Kavitäten und diese Kavitäten miteinander verbindende, für die Analyse benötigte Flüssigkeiten aufgrund des Kapillareffekts transportierende Kanäle angeordnet sind, wobei mindestens eine dieser Kavitäten eine Reaktionskammer ist.

Mikrofluidische Bauteile nutzen kapillare, hydrophile und hydrophobe Kräfte, um flüssige Stoffe ohne Pumpen in einem System bewegen zu können. Dazu sind Verzweiger und Mischer notwendig. Bei den heute bestehenden Systemen gibt es jedoch keine Möglichkeit, zwei Flüssigkeitsströme auf einem Fluidik-Chip sich kreuzen zu lassen, ohne dass eine Vermischung stattfindet.

Bei den in Rede stehenden Chips muss jedoch beispielsweise eine zu untersuchende Flüssigkeit (Blut) erst in einer Reaktionskammer mit einer Analyseflüssigkeit zusammengebracht werden, um anhand der zu beobachtenden Reaktion Aussagen über den zu untersuchenden Stoff machen zu können.

Der Mikrofluidik-Chip muss daher so aufgebaut sein, dass die die Flüssigkeit transportierenden Kanäle sowie die Kavitäten, aus denen die Flüssigkeiten transportiert werden vollkommen getrennt voneinander sind.

Aus dem Internet-Zitat:
URL:http://www.microtec-d.com/media/a homepage/a01 startseite/produkte biogentechnik.pd f "RMPD-Systeme für die Biogentechnik", siehe den Absatz "mikrofluidisches Lap-on-a-Chip" ist ein Mikrofluidik-Chip mit Mikrokapillaren und Kammern bekannt, wie er im Oberbegriff des Patentanspruchs 1 offenbart ist.

Des weiteren ergibt sich ein derartiger Mikrofluidik-Chip aus der US-B1- 6 213 151, der allerdings eine andere technische Verwendung findet.

Schließlich ist aus der WO00/58724 eine Vorrichtung zur Durchführung chemischer Nachweisreaktionen bekannt.

Demgegenüber ist der erfindungsgemäße Mikrofluidik-Chip durch einen schichtweisen Aufbau aus Licht aushärtendem hydrophilem Kunststoffmaterial anhand eines 3-D-Schichtmodells und eine Abdeckschicht aus einem hydrophoben Material gekennzeichnet, wobei in dem aus hydrophilem Material aufgebauten Schichtkörper aus verschiedenen Kavitäten kommende, kreuzungsfrei verlaufende Kanäle in der mindestens einen Reaktionskammer münden.

Dieser schichtweise Aufbau ist eine bekannte generative Technologie, bei der beispielsweise zwischen zwei Platten eine Licht aushärtende Flüssigkeit aufgrund ihrer Oberflächenspannung gehalten wird. Eine dieser Platten ist durchlässig für elektromagnetische Wellen. Beschrieben wird das Verfahren in der DE-PS 44 20 996.

Das Modell des zu erstellenden Körpers ist beispielsweise in einem Computer ebenfalls in virtuellen Schichten gespeichert, die nach und nach abgerufen werden und durch schichtweises Auseinanderfahren der beiden Platten und Nachströmen frischen Materials extrem dünne Schichten erzeugt werden können, die es ermöglichen, dass extrem genau und extrem kleine Strukturen erzeugt werden können, wie z.B. die Kavitäten, die Kanäle sowie auch Kavitäten, die durch Stege teilweise überbrückt werden. Auf diese Weise ist es möglich, dass zum einen die Kavitäten streng voneinander getrennt sind und zum anderen ebenfalls die die verschiedenen Flüssigkeiten transportierenden Kanäle.

Gemäß der weiteren Merkmalskombination des Anspruchs 1 ist vorgesehen, dass in dem Chip eine zentrale, von der hydrophoben Schicht abgedeckte Kavität generiert ist, die von einer ringförmig ausgebildeten Kavität umgeben ist, die ebenfalls von der hydrophoben Schicht abgedeckte, durch Stege voneinander getrennte Öffnungen aufweist, von denen jeweils ein Kanal zu einer der Öffnung zugeordneten Reaktionskammer, welche mit anderen sternförmig um die zentrale Kavität und um die Ringkavität angeordnet ist, führt, während von der zentralen Kavität in der Oberfläche der Stege geführte, die Ringkavität überbrückende Kanäle zur jeweils zugeordneten Reaktionskammer führen, wobei die von der Ringkavität sowie von der zentralen Kavität abgehenden Kanäle in senkrecht in den Wandungen der Kavitäten aufsteigende, zum Innenraum der Kavitäten hin offene Rinnen übergehen.

Dabei bietet die angewandte Methode des generativen bzw. schichtweisen Aufbaus die Gewähr, dass die Kanten der Kanäle an den Stellen, wo sie aus der Horizontalen in die Vertikale übergehen, extrem scharf ausgeführt werden können, so daß bei dem hydrophilen Material der Kapillareffekt sehr ausgeprägt ist.

Schließlich ist gemäß Anspruch 1 vorgesehen, dass die senkrecht in den Wandungen angeordneten Kanäle an ihrem kavitätsbodenseitigen Ende mit der Bodenfläche einen spitzen Winkel bilden.

D.h., dass an dieser Stelle der senkrecht nach unten verlaufende Kanal ein kurzes Stück aus der Kavität zurücktritt, eine Ausführungsform, die ebenfalls durch normale formgebende Verfahren nicht möglich ist. Für die Optimierung des Kapillareffekts ist eine derartige Ausgestaltung jedoch wesentlich.

Zur weiteren Optimierung des Kapillareffekts ist es wünschenswert, dass die Kanäle bzw. die Kavitäten durch eine hydrophobe Schicht abgedeckt sind.

Diese hydrophobe Schicht wird gemäß Anspruch 2 derart erzeugt, dass zunächst eine Folie aus einer oder mehreren Schichten aus Licht aushärtendem Kunststoffmaterial generiert wird, wobei die letzte Schicht lediglich teilpolymerisiert wird. Die Polymerisation erfolgt bekanntermaßen durch Belichtung mit elektromagnetischen Wellen.

Die so erzeugte Folie wird mit der teilpolymerisierten Schicht auf den zuvor generierten Mikrofluidik-Chip aufgelegt und dann wird die bislang lediglich teilpolymerisierte Schicht durch polymerisiert, so dass der Chip eine monolithische Struktur erhält.

Auf diese Weise kann auf Klebstoffe etc. verzichtet werden, die die feinen Kanäle beim Abdecken zusetzen können.

Um große Stückzahlen des in Rede stehenden Mikrofluidik-Chips erzeugen zu können, ist vorgesehen, dass die Folie kontinuierlich zwischen mindestens einem Walzenpaar erzeugt wird, wobei das Licht aushärtende Material zwischen den beiden Walzen angeordnet ist, von denen eine die zur Aushärtung dienende Beleuchtungseinrichtung aufweist und die so erzeugte Abdeckschicht ebenfalls im kontinuierlichen Verfahren auf die in großer Stückzahl erzeugten Mikrofluidik-Chips aufgelegt und durchpolymerisiert wird.

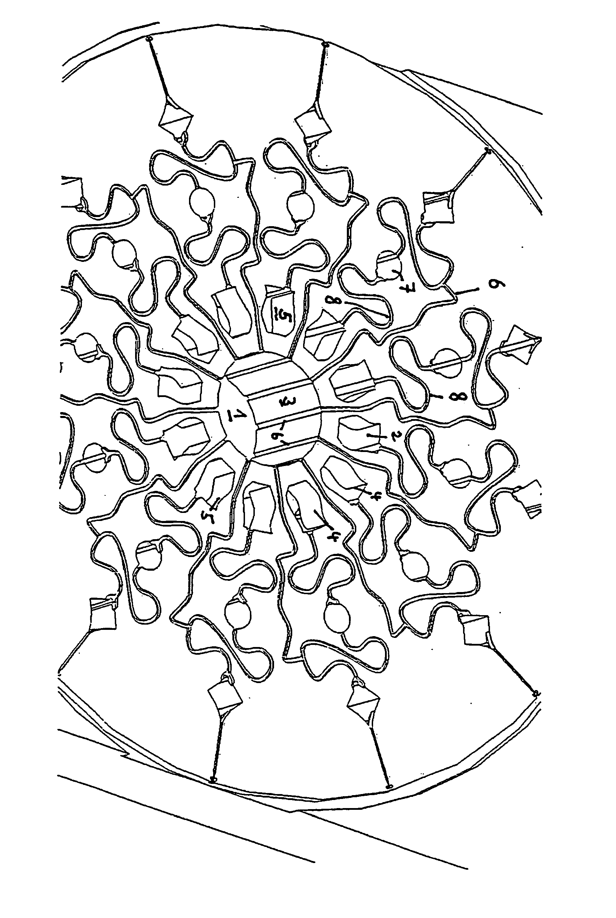

In der beigefügten Zeichnung ist im Ausschnitt und in stark vergrößerter Darstellung ein Mikrofluidik-Chip gezeigt, bei dem jedoch die Abdeckschicht weggelassen worden ist.

Der Chip weist eine zentrale Kavität 1 auf, die von einer ringförmigen Kavität 2 umgeben ist und durch die Wand 3 von dieser getrennt ist. Die ringförmige Kavität 2 weist sternförmig angeordnete Öffnungen 4 auf, die von Stegen 5 voneinander getrennt sind.

Vom Boden der zentralen Kavität 1 steigen senkrecht Kanäle 6 auf, die über die Stege 5 bis zu einer weiteren Kavität 7 geführt sind. Die Kavität 7 ist beispielsweise eine Reaktionskammer.

In dieser Kammer reagiert eine aus der zentralen Kavität 1 über die Kanäle 6 zugeführte Flüssigkeit mit beispielsweise einer Analyseflüssigkeit, die aus der ringförmigen Kavität 2 über Kanäle 8 zugeführt wird. Der Kanal 8 steigt vom Boden der ringförmigen Kavität 2 in der Wand dieser Kavität nach oben und wird dann kreuzungsfrei vom Kanal 6 zur Kavität 7 geführt.

Der Transport der Flüssigkeiten kommt durch die Kapillarwirkung des hydrophilen Materials, aus dem der Mikrofluidik-Chip besteht, zustande. Hierbei ist wesentlich, dass zum einen das nicht dargestellte Abdeckmaterial hydrophob ausgebildet ist und dass zum anderen die Kanten der Kanäle an den Stellen des Übergangs von der Vertikalen zur Horizontalen extrem scharf ausgebildet sind. Das betrifft zum einen die Kanten an denen die senkrecht aufsteigenden Kanäle 6 und 8 aus den jeweiligen Kavitäten 1 und 2 zur Chip-Oberfläche führen als auch die Kanten, an denen diese senkrechten Kanäle auf dem Boden der jeweiligen Kavität auftreffen. An diesen Stellen sind die Kanäle ein Stück in die Wand hineingeführt und bilden hier einen spitzen Winkel mit der Bodenfläche.

Derartige Chips werden beispielsweise vom Hersteller in dem Ringraum 2 mit einer Analyseflüssigkeit gefüllt. Zur Untersuchung wird das zu untersuchende Material, beispielsweise Blut oder Bestandteile von Blut, mittels einer Pipette oder Spritze durch die hier nicht dargestellte Abdeckschicht in die zentrale Kavität 1 eingefüllt. Aufgrund der Kapillarkräfte gelangen dann die beiden Flüssigkeiten in die Kammer 7. Anhand der dort stattfindenden Reaktion können Aussagen über den zu untersuchenden Stoff gemacht werden.

## Patentansprüche

1. Mikrofluidik-Chip für biologische, chemische und medizinische Analytik, in dem Kavitäten (1, 2) und diese Kavitäten miteinander verbindende, für Analyse und Synthese benötigte Flüssigkeiten aufgrund des Kapillareffekts transportierende Kanäle (6, 8) angeordnet sind, wobei mindestens eine der Kavitäten eine Reaktionskammer (7) ist, **gekennzeiohnet durch** einen schichtweisen Aufbau aus Licht aushärtendem, hydrophilem Kunststoffmaterial anhand eines Drei-D-Schichtmodells und eine Abdeckschicht aus einem hydrophobem Material, wobei in dem aus hydrophilem Material aufgebauten Schichtkörper aus verschiedenen Kavitäten (1, 2) kommende, kreuzungsfrei verlaufende Kanäle (6, 8) in die mindestens einen Reaktionskammer (7) münden, wobei in dem Chip eine Zentrale, von der hydrophoben Schicht abgedeckte Kavität (1) generiert ist, die von einer ringförmig ausgebildeten Kavität (2) umgeben ist, die ebenfalls von der hydrophoben Schicht abgedeckte, durch Stege (5) voneinander getrennte Öffnungen (4) aufweist, von denen jeweils ein Kanal (8) zu einer der Öffnung zugeordneten Reaktionskammer (7) führt, welche mit anderen Reaktionskammern sternförmig um die zentrale Kavität (1) und die Ringkavität (2) angeordnet ist, während von der zentralen Kavität (1) in der Oberfläche der Stege (5) geführte, die Ringkavität (2) überbrückende Kanäle (6) zur jeweils zugeordneten Reaktionskammer (7) führen, wobei die von der Ringkavität (2) sowie von der zentralen Kavität (1) abgehenden Kanäle (6, 8) in senkrecht in den Wandungen der Kavitäten (1,2) aufsteigende, zum Innenraum der Kavitäten (1,2) hin offene Rinnen übergehen, und wobei die senkrecht in den Wandungen angeordneten Kanäle (6, 8) an ihrem kavitätsbodenseitigen Ende mit der Bodenfläche einen spitzen Winkel bilden.

2. Mikrofluidik-Chip nach Anspruch 1,
**dadurch gekennzeichnet,**
**dass** die Abdeckschicht derart aufgebaut ist, dass zunächst eine Folie aus einer oder mehreren Schichten aus Licht aushärtendem Kunststoffmaterial generiert ist, wobei die letzte Schicht lediglich teilpolymerisiert wird, wonach die so entstandene Folie mit der teilpolymerisierten Schicht auf den zuvor generierten Mikrofluidik-Chip aufgelegt und die bislang lediglich teilpolymerisierte Schicht durchpolymerisiert wird, so dass der Chip eine monolithische Struktur erhält.

## Claims

1. Microfluidic chip for biological, chemical and medicinal analysis, in which cavities (1, 2) and channels (6, 8) which connect these cavities to one another and which use the capillary effect to transport liquids required for carrying out analysis and synthesis, are arranged inside said microfluidic chip, at least one of the cavities being a reaction chamber (7), **characterised by** a layered construction made of light-curing hydrophilic plastic material based on a 3-D layer model and a covering layer made of a hydrophobic material, while in the layered body made of hydrophilic material, channels (6, 8) which do not intersect, coming from different cavities (1, 2) lead to the at least one reaction chamber (7), while in the chip a central cavity (2) covered by the hydrophobic layer (1) is generated, which is surrounded by an annular cavity which also has openings (4) covered by the hydrophobic layer and separated from each other by webs (5), from each of which a channel (8) leads to a reaction chamber (7) assigned to the opening, which is arranged with other reaction chambers in the form of a star about the central cavity (1) and the annular cavity (2), while channels (6) leading from the central cavity (1) into the surface of the webs (5), bridging the annular cavity (2) lead to respectively assigned reaction chambers (7), while the channels (6, 8) outgoing from the annular cavity (2) and from the central cavity (1) graduate into grooves rising perpendicularly in the walls of the cavities (1, 2) which are open towards the interior of the cavities (1, 2) and in which the channels (6, 8) arranged perpendicularly in the walls form a vertical angle with the floor area at their cavity base ends.

2. Microfluidic chip according to claim 1,
**characterised in**
**that** the cover layer is constructed such that firstly a foil made from one or more layers of light-curing plastic material is generated, while the final layer is only partially polymerised, after which the foil thus produced is laid on the previously generated microfluidic chip and the previously only partially-polymerised layer is fully polymerised, so that the chip is given a monolithic structure.

## Revendications

1. Puce microfluidique pour analyses biologiques, chimiques et médicales, dans laquelle sont disposées des cavités (1, 2) et des canaux (6, 8) reliant entre elles ces cavités et transportant des liquides, nécessaires à l'analyse et à la synthèse, par effet de capillarité, l'une au moins des cavités étant une chambre de réaction (7), **caractérisée par** une construction stratifiée en une matière plastique hydrophile, durcissant à la lumière, à l'aide d'un modèle stratifié en trois dimensions, et une couche de couverture en un matériau hydrophobe, dans le corps stratifié, constitué d'un matériau hydrophile, des canaux (6, 8), s'étendant sans se croiser et provenant de différentes cavités (1, 2), débouchent dans la au moins une chambre de réaction (7), dans la puce est générée une cavité (1) centrale, recouverte par la couche hydrophobe, laquelle cavité (1) est entourée par une cavité (2) réalisée en forme d'anneau qui présente également des orifices (4), séparés les uns des autres par des cloisons (5) et recouverts par la couche hydrophobe, de chaque orifice un canal (8) menant à une chambre de réaction (7) associée à l'orifice, laquelle est disposée avec d'autres chambres de réaction, en étoile, autour de la cavité centrale (1) et la cavité annulaire (2), tandis que des canaux (6), franchissant la cavité annulaire (2) et guidés depuis la cavité centrale (1) dans la surface des cloisons (5), mènent à des chambres de réaction (7) associées à chacun d'eux, les canaux (6, 8), partant de la cavité annulaire (8) ainsi que de la cavité centrale (1), se prolongeant dans des rigoles montant verticalement dans les parois des cavités (1, 2) et ouvertes vers le volume intérieur des cavités (1, 2), et les canaux (6, 8), disposés verticalement dans les parois, formant, à leur extrémité côté fond de cavité, un angle aigu avec la surface du fond.

2. Puce microfluidique selon la revendication 1,
**caractérisée**
**en ce que** la couche de couverture est constituée de manière que soit générée d'abord une feuille constituée d'une ou de plusieurs couches d'une matière plastique durcissant à la lumière, la dernière couche étant seulement partiellement polymérisée, après quoi la feuille ainsi obtenue avec la couche partiellement polymérisée est placée sur la puce microfluidique générée précédemment et la couche, jusqu'alors seulement partiellement polymérisée, est polymérisée totalement, ce qui fait que la puce obtient une structure monolithique.
